# EUROPÄISCHE PATENTANMELDUNG

(11) **EP 3 524 213 A1**
(43) Veröffentlichungstag der Anmeldung: **14.08.2019**
(21) Anmeldenummer: 18155789.3
(22) Anmeldetag: 08.02.2018
(51) Int. Cl.: A61F 13/551, B65D 85/00, B65D 75/56, B65D 71/00

(54) **FOLIENVERPACKUNG FÜR HYGIENEPRODUKTE SOWIE VERWENDUNG DER FOLIENVERPACKUNG**

(71) Anmelder: Metsä Tissue Oyj, 02100 Espoo (FI)
(72) Erfinder: Schütz, Jürgen, 56593 Horhausen (DE); Scharfenstein, Gerd, 57639 Rodenbach-Udert (DE)
(74) Vertreter: Geskes, Christoph

(57) **Zusammenfassung**

Die Erfindung betrifft eine Folienverpackung (10) für Hygieneprodukte, umfassend zumindest einen Behälter (12) für Hygieneprodukte, wobei der Behälter (12) aus einem ersten Bogen (14) Kunststofffolie ausgebildet ist, wobei der erste Bogen (14) einen Zwischenraum (16) zur Anordnung von Hygieneprodukten umspannt, wobei der Behälter (12) eine von Zwischenraum (16) abgewandte Außenfläche (18) aufweist, wobei der Behälter (12) zumindest einen Behälterbefestigungsabschnitt (20) aufweist, an dem ein erster Abschnitt (22) des ersten Bogens (14) an einem zweiten Abschnitt (24) des ersten Bogens (14) befestigt ist, und zumindest einen Haltegriff (30), wobei der Haltegriff (30) aus einem zweiten Bogen (32) Kunststofffolie ausgebildet ist, wobei der Haltegriff (30) mindestens eine erste Grifföffnung (34) aufweist, und wobei der Haltegriff (30) zumindest einen Griffbefestigungsabschnitt (36) aufweist, an dem der zweite Bogen (32) an der Außenfläche (18) des Behälters (12) befestigt ist.

## Beschreibung

Die Erfindung betrifft eine Folienverpackung für Hygieneprodukte sowie eine Verwendung der Folienverpackung.

Folienverpackungen, insbesondere für Hygieneprodukte wie Küchenrollen, Toilettenpapier, Servietten, Taschentücher, Windeln und Binden, weisen, um einen einfachen Transport der Folienverpackung mit dem jeweiligen Hygieneprodukt zu ermöglichen, einen Haltegriff auf. Die Herstellung von einer Folienverpackung mit Griff führt derzeit zu einer Verteuerung der Folienverpackung aufgrund einer aufwendigen Herstellung. Zum Teil werden Griffe und Halteriemen bei bekannten Folienverpackungen vom Verbraucher übersehen, da sich diese nicht optisch vom Rest der Verpackung absetzen und beispielsweise sehr eng an der Verpackung anliegen.

Es ist daher die Aufgabe der vorliegenden Erfindung, eine verbesserte Folienverpackung, insbesondere mit einem für den Verbraucher leicht zu erkennenden Griff, für Hygieneprodukte bereitzustellen, die besonders einfach und kostengünstig herstellbar ist und daher eine besonders kostengünstige Verpackung von Hygieneprodukten ermöglicht.

Diese Aufgabe wird vorliegend gelöst durch die nachfolgend beschriebene erfindungsgemäße Folienverpackung und durch die nachfolgend beschriebene erfindungsgemäße Verwendung der Folienverpackung.

Die erfindungsgemäße Folienverpackung für Hygieneprodukte umfasst zumindest einen Behälter für Hygieneprodukte, wobei der Behälter aus einem ersten Bogen Kunststofffolie ausgebildet ist, wobei der erste Bogen einen Zwischenraum zur Anordnung von Hygieneprodukten umspannt, wobei der Behälter eine vom Zwischenraum abgewandte Außenfläche aufweist, wobei der Behälter zumindest einen Behälterbefestigungsabschnitt aufweist, an dem ein erster Abschnitt des ersten Bogens an einem zweiten Abschnitt des ersten Bogens befestigt ist und zumindest einen Haltegriff, wobei der Haltegriff aus einem zweiten Bogen Kunststofffolie ausgebildet ist, wobei der Haltegriff mindestens eine erste Grifföffnung aufweist und wobei der Haltegriff zumindest einen Griffbefestigungsabschnitt aufweist, an dem der zweite Bogen an der Außenfläche des Behälters befestigt ist.

Die erfindungsgemäße Folienverpackung hat den Vorteil, dass der Griff auf einen bestehenden Behälter einer Folienverpackung von außen angebracht werden kann. Das Herstellungsverfahren des Behälters muss nicht verändert werden. Hierbei sind sowohl Verpackungen des Typs Flachfolie als auch des Typs Beutel herstellbar. Insbesondere sind beide Typen mittels des gleichen Maschinentyps einer Flachfolienmaschine herstellbar. Daher ist die erfindungsgemäße Folienverpackung besonders einfach und kostengünstig herstellbar. Dadurch, dass der Griff erfindungsgemäß außen an die Verpackung angebracht wird, kann der Griff von der restlichen Verpackung hervorstehen und derart besonders leicht vom Verbraucher als Griff erkannt und genutzt werden.

Wird im Rahmen der Erfindung der Begriff "etwa" oder "im Wesentlichen", insbesondere in Bezug auf Werte oder Wertebereiche, verwendet, ist hierunter dasjenige zu verstehen, was der Fachmann in dem gegebenen Zusammenhang als fachmännisch üblich ansehen wird. Insbesondere sind Abweichungen der angegeben Werte oder Wertebereiche von +/- 10%, bevorzugt +/- 5%, weiter bevorzugt von +/- 2%, von den Begriffen "etwa" und "im Wesentlichen" umfasst.

Unter einem Behälter wird erfindungsgemäß ein Gebilde verstanden, in dem Hygieneprodukte angeordnet werden können. Der Behälter kann beispielsweise beutelförmig oder paketartig ausgebildet sein. Der Behälter kann beispielweise eine im Wesentlichen quaderförmige Gestalt aufweisen. Der Behälter kann im Wesentlichen quaderförmig mit zwei Stirnseiten und mit zumindest zwei, vorzugsweise vier, Längsseiten ausgebildet sein. Hierbei beeinflussen die verpackten Hygieneprodukte die Form des Behälters. Bei Küchenrollen kann der Behälter beispielsweise zwei halbrunde beziehungsweise gewölbte Stirnseiten und zwei Paar flache Längsseiten aufweisen. Im Sinne der Erfindung wird auch hierunter ein im Wesentlichen quaderförmiger Behälter verstanden. Der erste Abschnitt und der zweite Abschnitt des ersten Bogens sind vorzugsweise an einer ersten Längsseite desselbigen ausgebildet. Der erste Bogen weist vorteilhafterweise einen dritten Abschnitt und einen vierten Abschnitt beziehungsweise ein zweites Paar Abschnitte auf, die an einer zweiten Längsseite des Bogens ausgebildet sind. Der Behälter ist insbesondere dadurch herstellbar, dass der erste Bogen Kunststofffolie, um die zu verpackenden Hygieneprodukte herumgeschlagen beziehungsweise gefaltet wird und die Abschnitte aneinander befestigt werden, so dass eine Art Folien-Paket entsteht. Der erste Bogen ist beispielsweise derart gefaltet, dass der erste und der zweite Abschnitt an der Oberseite des Behälters zumindest teilweise übereinander in dem Behälterbefestigungsabschnitt angeordnet sind. Die Abschnitte sind aneinander, insbesondere mittels eines Kunststoffschweißverfahrens, befestigt. Denkbar sind auch Klebeverfahren. Die Unterseite des Behälters kann wie die beschriebene Oberseite ausgebildet sein. An der Unterseite kann ein weiterer Behälterbefestigungsabschnitt ausgebildet sein, an dem der dritte und vierte Abschnitt des ersten Bogens aneinander befestigt sind.

Der erste Bogen ist besonders bevorzugt aus einem kunststoffschweißbaren Material ausgebildet. Besonders bevorzugt ist der Bogen aus Polyethylen ausgebildet. Der erste Bogen kann auch Polypropylen, Polyvinylalkohol, Polylactide und Ähnliches umfassen. Der zweite Bogen Kunststofffolie ist vorzugsweise aus demselben Material wie der erste Bogen Kunststofffolie ausgebildet. Der erste und der zweite Bogen Kunststofffolie können jeweils eine Dicke von etwa 0,02mm bis etwas 0,15mm aufweisen.

Um eine besonders stabile Verankerung des Griffbefestigungsabschnitts an dem Behälter zu realisieren, ist der Griffbefestigungsabschnitt besonders bevorzugt zumindest teilweise auf dem Behälterbefestigungsabschnitt angeordnet. Denn der Behälterbefestigungsabschnitt weist zumindest teilweise eine erhöhte Materialstärke beziehungsweise Dicke auf, da dort mehrere Folienlagen beziehungsweise der erste und der zweite Abschnitt des ersten Bogens Kunststofffolie aneinander befestigt sind. Der Behälterbefestigungsabschnitt bildet daher ein besonders stabiles Fundament für den Griffbefestigungsabschnitt. Um eine besonders einfache und kostengünstige Herstellung zu ermöglichen, weist der Haltegriff vorzugsweise lediglich einen einzigen Griffbefestigungsabschnitt auf. Alternativ kann der Haltegriff beispielsweise zumindest zwei beabstandete Griffbefestigungsabschnitte aufweisen, so dass der Haltegriff beispielsweise henkelförmig beziehungsweise riemenförmig oder schlaufenförmig ausgebildet ist. Der zweite Bogen kann hierfür insbesondere mehrlagig sein, beispielweise durch eine oder mehrere Faltungen des zweiten Bogens. Die erste Grifföffnung kann dann alternativ beispielsweise durch den unbefestigten Bereich zwischen den beabstandeten Griffbefestigungsabschnitten bereitgestellt werden. Besonders bevorzugt ist die erste Grifföffnung jedoch als Aussparung im zweiten Bogen ausgebildet.

Um für eine besonders robuste Anordnung des Haltegriffs einen möglichst langen Griffbefestigungsabschnitt an dem Behälter zu realisieren, ist der Griffbefestigungsabschnitt besonders bevorzugt auf einer Längsseite, besonders bevorzugt auf einer schmalen Längsseite, des Behälters angeordnet. Hierdurch ist die Verpackung auch besser stapelbar.

Der Griffbefestigungsabschnitt kann auch auf einer Stirnseite des Behälters angeordnet sein. Der Behälter kann an der jeweils gegenüberliegenden Stirnseite eine Entnahmeöffnung aufweisen. Derart kann der Verbraucher die Verpackung "vertikal" am Griff aufhängen. Bei Entnahme eines Hygieneprodukts aus der dann unterseitig angeordneten Entnahmeöffnung rutschen die restlichen verpackten Hygieneprodukte nach und können bei Bedarf besonders einfach entnommen werden. Dieser Effekt kann auch durch eine dezentrale Anordnung des Haltegriffs auf einer Längsseite erreicht werden. Der Haltegriff kann beispielsweise in einem zu einer Stirnseite benachbarten Bereich einer Längsseite angeordnet sein.

Der Griffbefestigungsabschnitt ist vorzugsweise im Wesentlichen streifenförmig ausgebildet, um eine einfache automatisierte Herstellung zu ermöglichen. Alternativ oder ergänzend kann der Behälterbefestigungsabschnitt auch streifenförmig ausgebildet sein. Unter streifenförmig werden in Sinne der Erfindung auch linienförmige Ausbildungen verstanden, beispielsweise solche bestehend aus nur einer Schweißnaht oder einer Linie aus mehreren Schweißpunkten. Besonders bevorzugt weisen der Griff- und/oder der Befestigungsabschnitt die Form eines geraden Streifens auf. Es ist auch denkbar, dass der "Streifen" eine andersförmige Kontur ausweist, beispielsweise eine ellipsenförmige Kontur. Der "Streifen" kann auch zumindest teilweise die Form einer gekrümmten Linie aufweisen.

Vorzugsweise ist der Behälterbefestigungsabschnitt und/oder der Griffbefestigungsabschnitt mittels eines Kunststoffschweißverfahrens hergestellt. Hierdurch ist auf einfache Weise eine feste Verbindung der jeweiligen Abschnitte möglich. Beispielsweise kann der Behälterbefestigungsabschnitt und/oder der Griffbefestigungsabschnitt zumindest eine Kunststoffschweißnaht und oder mehrere Kunststoffschweißpunkte umfassen. Der Behälterbefestigungsabschnitt und/oder der Griffbefestigungsabschnitt wird beispielsweise dadurch hergestellt, dass eine Vorrichtung zum Kunststoffschweißen in Anlage mit dem jeweiligen Befestigungsabschnitt gebracht wird und eine oder mehrere Kunststoffschweißnähte und/oder mehrere Kunststoffschweißpunkte im Bereich des jeweiligen Befestigungsabschnitts ausgebildet werden. Alternativ oder ergänzend kann auch ein Klebeverfahren verwendet werden.

Um die Dicke beziehungsweise Materialstärke des Behälterbefestigungsabschnitts zu erhöhen und um hierdurch eine besonders stabile Befestigung des Griffbefestigungsabschnitts zu ermöglichen, umfasst der erste Bogen zumindest eine Faltung, wobei die Faltung zumindest teilweise im Behälterbefestigungsabschnitt angeordnet ist. Durch die erhöhte Dicke ist auch eine besonders sichere Befestigung des ersten und des zweiten Abschnitts des ersten Bogens Kunststofffolie zur Ausbildung des Behälters realisierbar. Der erste Bogen weist vorzugsweise zumindest vier Faltungen auf, die im Behälterbefestigungsabschnitt angeordnet sind. Im ersten und zweiten Abschnitt des ersten Bogens sind vorzugsweise ist jeweils ein Paar Faltungen ausgebildet. Alternativ oder ergänzend kann auch eine Folie mit erhöhter Dicke für den Behälter als erster Bogen Kunststofffolie verwendet werden.

Besonders bevorzugt ist der erste Bogen und/oder der zweite Bogen im Wesentlichen rechteckig ausgebildet, um eine besonders einfache Herstellung beispielsweise durch Zuschnitt von Rollmaterial zu ermöglichen. Denkbar sind auch andere Formen. Insbesondere der Haltegriff beziehungsweise der zweite Bogen kann verschiedenste Formen aufweisen. Die Ränder des ersten und/oder des zweiten Bogens können gekrümmte Abschnitte umfassen. Beispielsweise kann der zweite Bogen zumindest einen halbkreisförmigen Abschnitt aufweisen, um einen einsprechenden bogenförmigen Griff auszubilden.

Um sich besonders deutlich von dem Rest der Verpackung abzusetzen, kann der Haltegriff beziehungsweise der zweite Bogen farbig ausgebildet sein, insbesondere einfarbig oder mehrfarbig. Der Haltegriff kann beispielsweise einen Motivdruck aufweisen. Alternativ kann der Haltegriff zumindest teilweise transparent ausgebildet sein.

Die erste Grifföffnung des Haltegriffs ist bevorzugt als länglicher Schlitz ausgebildet, um das Durchgreifen einer ganzen Hand zu ermöglichen. Der Griff kann eine oder mehrerer einzelne oder zumindest teilweise zusammenhängende Öffnungen für einzelne Finger, beispielsweise in Form von runden Aussparungen, umfassen. Durch mehrere einzelne kleinere Grifföffnungen anstelle einer einzigen größeren Grifföffnung wird die Last im Griff besonders gleichmäßig verteilt. Hierdurch wird ein besonders langlebiger Griff bereitgestellt. Der Haltegriff kann zumindest eine zweite Grifföffnung aufweisen. Besonders bevorzugt sind die erste Grifföffnung und die zweite Grifföffnung in Bezug auf den Griffbefestigungsabschnitt im Wesentlichen symmetrisch in Bezug zueinander auf dem zweiten Bogen ausgebildet. Durch die symmetrische Ausbildung ist vorteilhafterweise ein Durchgriff durch beide Grifföffnungen möglich. Die zweite Grifföffnung ist daher auch bevorzugt deckungsgleich zur ersten Grifföffnung ausgebildet. Dies ist besonders für einen wie folgt beschriebenen mehrlagig ausgebildeten Haltegriff vorteilhaft.

Um eine Mehrlagigkeit des Haltegriffs zu realisieren beziehungsweise um vorteilhafterweise einen Haltegriff mit zumindest zwei überlappenden Lagen Kunststofffolie bereitzustellen, wobei eine einfache Herstellung beibehalten wird, weist der zweite Bogen besonders bevorzugt zumindest eine erste unbefestigte Kante und eine zweite unbefestigte Kante auf, wobei der Griffbefestigungsabschnitt zwischen den beiden unbefestigten Kanten ausgebildet ist. Diese Kanten sind vorteilhafterweise Kanten des zweiten Bogens, die, insbesondere bei einer rechteckigen Ausführung des zweiten Bogens, nicht zueinander benachbart sind. Durch die Mehrlagigkeit wird ein ungewolltes Durchreißen des Haltegriffs entgegengewirkt. Alternativ oder ergänzend kann auch eine Folie mit erhöhter Dicke für den Griff als zweiter Bogen Kunststofffolie verwendet werden.

Besonders bevorzugt ist der Griffbefestigungsabschnitt im Wesentlichen mittig zwischen den zwei unbefestigten Kanten des zweiten Bogens angeordnet. Derart sind die entstehenden zwei Lagen Kunststofffolie des Haltegriffes vorteilhafterweise überlappend beziehungsweise im Wesentlichen deckungsgleich ausgebildet. Um einen Durchgriff zu ermöglichen, ist bevorzugt an der zweiten Lage des zweiten Bogens eine zweite Grifföffnung ausgebildet. Die zweite Grifföffnung und die erste Grifföffnung sind vorzugsweise zueinander im Wesentlichen symmetrisch in Bezug zu dem Begriffsbefestigungsabschnitt auf dem zweiten Bogen ausgebildet. Die erste und die zweite Grifföffnung sind vorzugsweise schlitzförmig ausgebildet.

Alternativ kann der Griffbefestigungsabschnitt, beispielsweise aus fertigungstechnischen Gründen, den zweiten Bogen in eine erste Lage mit der unbefestigten ersten Kante und in eine zweite Lage mit der zweiten unbefestigten Kante unterteilen, wobei die Lagen nicht gleichgroß beziehungsweise nicht deckungsgleich sind. Die zweite Lage kann beispielsweise wesentlich schmaler als die erste Lage ausgebildet sein, so dass nur die erste Lage als Griff fungiert und daher die erste Grifföffnung an der ersten Lage ausgebildet ist. Die erste Grifföffnung ist vorzugsweise schlitzförmig ausgebildet.

Alternativ kann der Griffbefestigungsabschnitt derart angeordnet sein, dass der Bogen nur eine Lage aufweist. Der zweite Bogen weist beispielsweise zumindest eine befestigte Kante auf, wobei der Griffbefestigungsabschnitt an dieser Kante ausgebildet ist.

Gemäß einer ersten bevorzugten Ausführungsform weist die Folienverpackung einen Behälter auf, der im Wesentlichen paketartig ausgebildet ist. Der Behälter besteht aus einem ersten Bogen Kunststofffolie, der zu einem Paket gefaltet wurde, so dass der erste Bogen einen Zwischenraum zur Anordnung von Hygieneprodukten umspannt. Hierbei ist ein erster Abschnitt des ersten Bogens an einem zweiten Abschnitt des ersten Bogens befestigt. Der Behälter weist eine von dem Zwischenraum abgewandte Außenfläche auf. Der erste Bogen ist im Wesentlichen rechteckig ausgebildet. Der erste Abschnitt und der zweite Abschnitt des Bogens sind an einer Längsseite desselbigen ausgebildet. Der Bogen ist derart gefaltet, dass der erste und der zweite Abschnitt an der Oberseite des Behälters zumindest teilweise übereinander in dem Behälterbefestigungsabschnitt angeordnet sind. Der erste Bogen weist vorzugsweise zumindest vier Faltungen auf, die im Behälterbefestigungsabschnitt angeordnet sind. Im ersten und zweiten Abschnitt des ersten Bogens sind vorzugsweise jeweils ein Paar Faltungen ausgebildet. Die Unterseite des Behälters kann entsprechend wie die beschriebene Oberseite ausgebildet sein. An der Unterseite kann ein weiterer Behälterbefestigungsabschnitt ausgebildet sein, an dem ein dritter und ein vierte Abschnitt des ersten Bogens aneinander befestigt sind. Gemäß der ersten bevorzugten Ausführungsform weist die Folienverpackung einen Haltegriff auf, der auf dem oberseitigen Behälterbefestigungsabschnitt angeordnet ist, wobei der Haltegriff an der Außenfläche des Behälters befestigt ist. Der Haltegriff ist aus einem rechteckigen zweiten Bogen Kunststofffolie ausgebildet. Der zweite Bogen des Haltegriffs weist eine erste unbefestigte Kante und eine zweite unbefestigte Kante auf, wobei der Griffbefestigungsabschnitt zwischen den beiden unbefestigten Kanten ausgebildet ist. Diese Kanten des zweiten Bogens sind zueinander nicht benachbart. Der Griffbefestigungsabschnitt unterteilt den zweiten Bogen bei dieser bevorzugten Ausführungsform in eine erste Lage mit einer unbefestigten ersten Kante und in eine zweite Lage mit der zweiten unbefestigten Kante, wobei die Lagen nicht gleichgroß beziehungsweise nicht deckungsgleich sind. Die zweite Lage ist schmaler als die erste Lage ausgebildet, so dass nur die erste Lage als Griff fungiert und die erste Grifföffnung an der ersten Lage ausgebildet ist. Die erste Grifföffnung ist schlitzförmig ausgebildet.

Eine zweite bevorzugte Ausführungsform der erfindungsgemäßen Folienverpackung unterscheidet sich darin von der beschriebenen ersten bevorzugten Ausführungsform, dass diese einen andersartig ausgebildeten Haltegriff aufweist. Der Griffbefestigungsabschnitt des Haltegriffs dieser Ausführungsform ist mittig zwischen den zwei unbefestigten Kanten des zweiten Bogens des Haltegriffs angeordnet. Die erste und die zweite Lage bei diesem Haltegriff sind im Wesentlichen gleich breit ausgebildet. An der zweiten Lage des zweiten Bogens ist eine zweite schlitzförmige Grifföffnung ausgebildet. Die zweite Grifföffnung und die erste Grifföffnung sind zueinander im Wesentlichen symmetrisch in Bezug zu dem Griffbefestigungsabschnitt auf dem zweiten Bogen ausgebildet.

Um einen mehrlagigen Haltegriff in Form einer Schlaufe herstellen zu können, umfasst der zweite Bogen zumindest eine Faltung oder Biegung, wobei der Griffbefestigungsabschnitt von der Faltung oder Biegung beabstandet ausgebildet ist und zumindest eine erste Lage und eine zweite Lage des zweiten Bogens aneinander beziehungsweise an der Außenfläche des Behälters befestigt. Hierbei sind die zwei nicht befestigten Kanten die losen Enden des schlaufenförmigen Haltegriffs. Die Biegung beziehungsweise Faltung ist vorzugsweise mittig an dem zweiten Bogen angeordnet.

Alternativ können im Griffbefestigungsabschnitt zumindest eine oder zwei Kanten des zweiten Bogens angeordnet und befestigt sein. Der Griffbefestigungsabschnitt kann derart angeordnet sein, dass ein oder beide Enden des schlaufenförmigen Haltegriffs im Griffbefestigungsabschnitt angeordnet und befestig sind. Hierdurch ist eine besonders ansprechende Befestigung des Haltegriffs realisierbar.

Besonders bevorzugt weist der schlaufenförmige Haltegriff eine zweite Grifföffnung auf. Die erste und die zweite Grifföffnung sind vorzugsweise in Bezug auf den Griffbefestigungsabschnitt und der Biegung symmetrisch auf dem zweiten Bogen ausgebildet, um einen Durchgriff durch beide Öffnungen zu ermöglichen. Die zwei Grifföffnungen sind vorteilhafterweise an gegenüberliegenden Lagen des Haltegriffs ausgebildet. Die erste und die zweite Grifföffnung sind vorzugsweise schlitzförmig ausgebildet. Eine dritte bevorzugte Ausführungsform der Folienverpackung unterscheidet sich lediglich in Bezug auf den Haltegriff von den vorbeschriebenen Ausführungsformen. Der Haltegriff bei dieser bevorzugten Ausführungsform ist schlaufenförmig ausgebildet und weist eine Biegung beziehungsweise Faltung auf, die mittig an dem zweiten Bogen angeordnet ist. Der Griffbefestigungsabschnitt ist von der Biegung beabstandet ausgebildet und befestigt die erste Lage und die zweite Lage des zweiten Bogens an der Außenfläche des Behälters auf dem Behälterbefestigungsabschnitt. Der Haltegriff weist eine zweite Grifföffnung auf. Die zweite Grifföffnung und die erste Grifföffnung sind in Bezug auf den Griffbefestigungsabschnitt und der Biegung symmetrisch auf dem zweiten Bogen ausgebildet. Die Grifföffnungen sind schlitzförmig ausgebildet.

Gemäß einer besonders bevorzugten Weiterbildung weist der vorbeschriebene schlaufenförmige Haltegriff einen ersten und einen zweiten Griffbefestigungsabschnitt auf. Die beiden Griffbefestigungsabschnitte sind vorzugsweise voneinander und von der Biegung beziehungsweise Faltung beabstandet ausgebildet. Die beiden Griffbefestigungsabschnitte können derart angeordnet sein, dass diese jeweils eine Kante des zweiten Bogens an der Außenfläche des Behälters befestigen. Es ist auch möglich, dass die Griffbefestigungsabschnitte jeweils zwischen den jeweiligen Kanten des zweiten Bogens und der Biegung angeordnet sind, so dass die Kanten selbst unbefestigt sind.

Erfindungsgemäß wird die vorbeschriebene Folienverpackung zur Verpackung von einem einzelnen Hygieneprodukt oder mehreren Hygieneprodukten verwendet, wobei die Hygieneprodukte insbesondere Küchenrollen, Toilettenpapier, Servietten, Taschentücher, Windeln und/oder Binden umfassen.

Weitere vorteilhafte Ausgestaltungen gehen aus den nachfolgenden Zeichnungen hervor. Die in den Figuren der Zeichnungen dargestellten Ausführungsformen sind nicht beschränkend auszulegen. Vielmehr können dort beschriebene Merkmale untereinander und mit den zuvor beschriebenen Merkmalen zu weiteren Ausgestaltungen kombiniert werden. Gleiche Teile oder Teile mit gleicher Funktion können die gleichen Bezugszeichen aufweisen. Es zeigen:
- Fig. 1:: eine perspektivische Seitenansicht auf eine erfindungsgemäße Folienverpackung gemäß einer ersten Ausführungsform;
- Fig. 2:: eine Draufsicht auf die Folienverpackung gemäß Fig. 1;
- Fig. 3:: eine schematische Seitenansicht der Folienverpackung gemäß Fig. 1;
- Fig. 4:: eine perspektivische Seitenansicht der erfindungsgemäßen Folienverpackung gemäß einer zweiten Ausführungsform;
- Fig. 5:: eine perspektivische Seitenansicht einer dritten Ausführungsform der erfindungsgemäßen Folienverpackung; und
- Fig. 6:: eine perspektivische Seitenansicht einer vierten Ausführungsform der erfindungsgemäßen Folienverpackung.

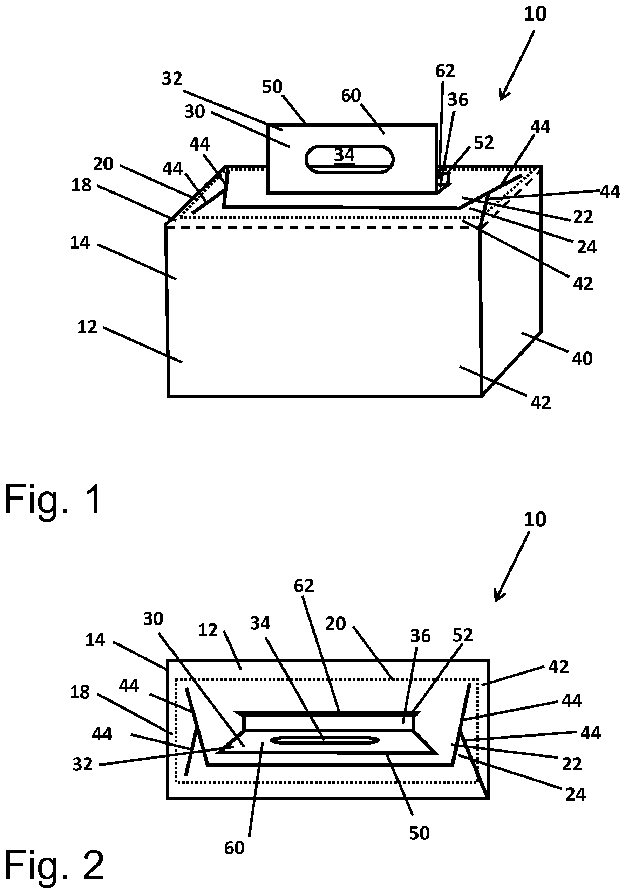

Fig. 1 zeigt, dass die Folienverpackung 10 gemäß der ersten Ausführungsform einen Behälter 12 für Hygieneprodukte wie Küchenrollen, Toilettenpapier, Servietten, Taschentücher, Windeln und/oder Binden umfasst. Der Behälter 12 ist im Wesentlichen quaderförmig beziehungsweise paketartig ausgebildet. Der Behälter 12 besteht aus einem ersten Bogen 14 Kunststofffolie, der, um die Hygieneprodukte zu beinhalten, zu einem Paket gefaltet wurde, so dass der erste Bogen 14 einen Zwischenraum 16 zur Anordnung von Hygieneprodukten umspannt. Hierbei ist ein erster Abschnitt 22 des ersten Bogens 14 an einem zweiten Abschnitt 24 des ersten Bogens 14 befestigt.

Der Behälter 12 weist eine von dem Zwischenraum 16 abgewandte Außenfläche 18 auf. Der erste Bogen 14 ist im Wesentlichen rechteckig ausgebildet. Der erste Abschnitt 14 und der zweite Abschnitt 24 des Bogens sind an einer Längsseite desselbigen ausgebildet. Der Bogen 14 ist derart gefaltet, dass der erste und der zweite Abschnitt 22, 24 an der Oberseite des Behälters zumindest teilweise übereinander in dem Behälterbefestigungsabschnitt 20 angeordnet sind beziehungsweise überlappen. Die nicht gezeigte Unterseite des Behälters 12 ist wie die beschriebene Oberseite ausgebildet. Der Behälterbefestigungsabschnitt 20 wird dadurch hergestellt, dass eine Vorrichtung zum Kunststoffschweißen in Anlage mit dem Behälterbefestigungsabschnitt 20 gebracht wird und nicht dargestellte Kunststoffschweißnähte im Bereich des Behälterbefestigungsabschnitts 20 ausbildet und dadurch der erste und der zweite Abschnitt 22, 24 aneinander befestigt werden. Durch die zumindest teilweise übereinander angeordneten Abschnitte 22, 24 des ersten Bogens 14 weist der Behälterbefestigungsabschnitt 20 eine erhöhte Dicke beziehungsweise Materialstärke auf. Die Folienverpackung 10 weist einen Haltegriff 30 auf, der auf dem Behälterbefestigungsabschnitt 20 angeordnet ist, wobei der Haltegriff 30 an der Außenfläche 18 des Behälters 12 befestigt ist.

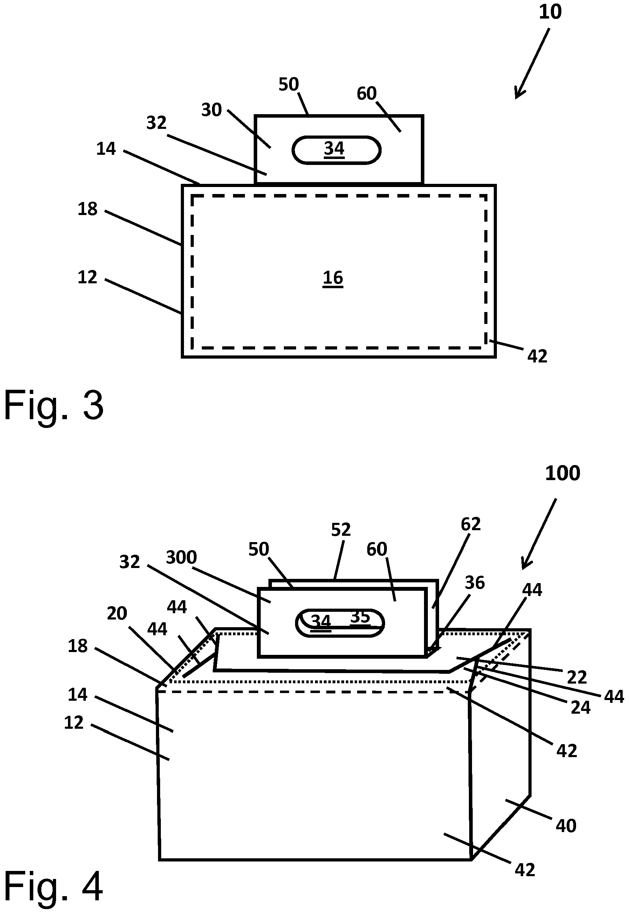

Fig. 2 und Fig. 3 zeigen, dass der Haltegriff 30 aus einem zweiten Bogen 32 Kunststofffolie ausgebildet ist, wobei der Bogen 32 rechteckig ausgebildet ist und eine erste Grifföffnung 34 aufweist. Die Grifföffnung 34 ist als länglicher Schlitz ausgebildet. Zur Befestigung des Haltegriffs 30 an dem Behälter weist der Haltegriff 30 einen streifenförmigen Griffbefestigungsabschnitt 36 auf.

Der Behälter 12 ist im Wesentlichen quaderförmig mit zwei Stirnseiten 40 und vier Längsseiten 42 ausgebildet, wobei der Griffbefestigungsabschnitt 36 auf der oberseitigen Längsseite 42 des Behälters angeordnet ist. Der Griffbefestigungsabschnitt 36 ist an der Außenfläche 18 des Behälters 12 am Behälterbefestigungsabschnitt 20 befestigt. Der erste Bogen 14 weist vier Faltungen 44 auf, die im Behälterbefestigungsabschnitt 20 angeordnet sind. Im ersten und zweiten Abschnitt 22, 24 ist jeweils ein Paar Faltungen 44 ausgebildet. Durch diese Faltungen 44 erhöht sich die Materialstärke im Behälterbefestigungsabschnitt 20 zusätzlich. Der zweite Bogen 32 weist eine erste unbefestigte Kante 50 und eine zweite unbefestigte Kante 52 auf. Der Griffbefestigungsabschnitt 36 ist zwischen den beiden unbefestigten Kanten 50, 52 des zweiten Bogens 32 ausgebildet. Der Griffbefestigungsabschnitt 36 unterteilt den zweiten Bogen 32 in eine erste Lage 60 mit der unbefestigten ersten Kante 50 und in eine zweite Lage 62 mit der zweiten unbefestigten Kante 52. Bei dem Griff 30 ist die zweite Lage 62 wesentlich schmaler als die erste Lage 60, so dass die erste Lage 60 als Griff fungiert. Die erste Grifföffnung 34 ist zwischen der ersten unbefestigten Kante 50 und dem Griffbefestigungsabschnitt 36 an der ersten Lage 60 ausgebildet.

Die in Fig. 4 gezeigte zweite Ausführungsform der erfindungsgemäßen Folienverpackung 100 unterscheidet sich von der ersten Ausführungsform, welche in den Fig. 1 bis 3 gezeigt ist, dadurch dass diese einen andersartig ausgebildeten Haltegriff 300 aufweist. Der Griffbefestigungsabschnitt 36 des Haltegriffs 300 ist mittig zwischen zwei unbefestigten Kanten 50, 52 des zweiten Bogens 32 des Haltegriffs 300 angeordnet. Die erste und die zweite Lage 60, 62 bei dem Haltegriff 300 sind daher im Wesentlichen gleich breit ausgebildet. Um einen Durchgriff zu ermöglichen, ist an der zweiten Lage 62 des zweiten Bogens 32 eine zweite Grifföffnung 35 ausgebildet. Die zweite Grifföffnung 35 und die erste Grifföffnung 34 sind zueinander im Wesentlichen symmetrisch in Bezug zu dem Griffbefestigungsabschnitt 36 auf dem zweiten Bogen 32 ausgebildet. Der Haltegriff 300 umfasst zwei deckungsgleiche Lagen 60, 62.

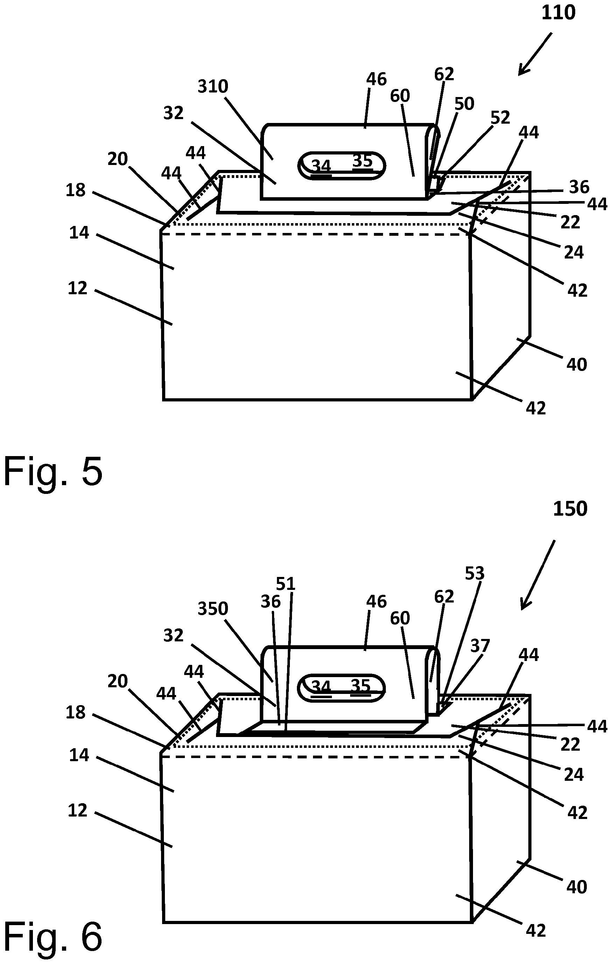

Die in Fig. 5 dargestellte dritte Ausführungsform der Folienverpackung 110 unterscheidet sich lediglich im Haltegriff 310 von den vorbeschriebenen Ausführungsformen. Der Haltegriff 110 ist schlaufenförmig ausgebildet und weist eine Biegung beziehungsweise Faltung 46 auf, die mittig an dem zweiten Bogen 32 angeordnet ist. Der Griffbefestigungsabschnitt 36 ist von der Biegung beziehungsweise Faltung 46 beabstandet ausgebildet und befestigt die erste Lage 60 und die zweite Lage 62 des zweiten Bogens 32 an der Außenfläche 18 des Behälters 12. Auch der Haltegriff 310 weist eine zweite Grifföffnung 35 auf. Die zweite Grifföffnung und die erste Grifföffnung 34, 35 sind in Bezug auf den Griffbefestigungsabschnitt 36 und der Biegung 46 symmetrisch auf dem zweiten Bogen 32 ausgebildet.

Die in Fig. 6 dargestellte vierte Ausführungsform der Folienverpackung 150 unterscheidet sich lediglich im Haltegriff 350 von den vorbeschriebenen Ausführungsformen. Wie der Haltegriff 110 ist auch der Haltegriff 150 schlaufenförmig ausgebildet und weist eine Biegung beziehungsweise Faltung 46 auf, die mittig an dem zweiten Bogen 32 angeordnet ist. Der Haltegriff 150 weist einen ersten und einen zweiten Griffbefestigungsabschnitt 36, 37 auf. Die beiden Griffbefestigungsabschnitte 36, 37 sind voneinander und von der Biegung beziehungsweise Faltung 46 beabstandet ausgebildet und befestigen jeweils eine Kante 51, 53 des zweiten Bogens 32 an der Außenfläche 18 des Behälters 12. Auch der Haltegriff 350 weist eine zweite Grifföffnung 35 auf. Die zweite Grifföffnung und die erste Grifföffnung 34, 35 sind in Bezug auf den Griffbefestigungsabschnitt 36 und der Biegung 46 symmetrisch auf dem zweiten Bogen 32 ausgebildet.

## Patentansprüche

1. Folienverpackung (10, 100, 110, 150) für Hygieneprodukte, umfassend zumindest einen Behälter (12) für Hygieneprodukte, wobei der Behälter (12) aus einem ersten Bogen (14) Kunststofffolie ausgebildet ist, wobei der erste Bogen (14) einen Zwischenraum (16) zur Anordnung von Hygieneprodukten umspannt, wobei der Behälter (12) eine von Zwischenraum (16) abgewandte Außenfläche (18) aufweist, wobei der Behälter (12) zumindest einen Behälterbefestigungsabschnitt (20) aufweist, an dem ein erster Abschnitt (22) des ersten Bogens (14) an einem zweiten Abschnitt (24) des ersten Bogens (14) befestigt ist, und zumindest einen Haltegriff (30, 300, 310, 350), wobei der Haltegriff (30, 300, 310, 350) aus einem zweiten Bogen (32) Kunststofffolie ausgebildet ist, wobei der Haltegriff (30, 300, 310, 350) mindestens eine erste Grifföffnung (34) aufweist, und wobei der Haltegriff (30, 300, 310, 350) zumindest einen Griffbefestigungs-abschnitt (36) aufweist, an dem der zweite Bogen (32) an der Außenfläche (18) des Behälters (12) befestigt ist.

2. Folienverpackung (10, 100, 110, 150) gemäß Anspruch 1, wobei der Griffbefestigungsabschnitt (36) zumindest teilweise auf dem Behälterbefestigungsabschnitt (20) angeordnet ist.

3. Folienverpackung (10, 100, 110, 150) gemäß Anspruch 1 oder 2, wobei der Haltegriff (30, 300, 310, 350) einen einzigen Griffbefestigungsabschnitt (36) aufweist.

4. Folienverpackung (10, 100, 110, 150) gemäß einem oder mehreren der vorhergehenden Ansprüche, wobei der Behälter (12) im Wesentlichen quaderförmig mit zwei Stirnseiten (40) und zumindest zwei Längsseiten (42) ausgebildet ist, wobei der Griffbefestigungs-abschnitt (36) auf einer Längsseite (42) des Behälters (12) angeordnet ist.

5. Folienverpackung (10, 100, 110, 150) gemäß einem oder mehreren der vorhergehenden Ansprüche, wobei der Behälterbefestigungsabschnitt (20) und/oder der Griffbefestigungsabschnitt (36) im Wesentlichen streifenförmig ausgebildet ist.

6. Folienverpackung (10, 100, 110, 150) gemäß einem oder mehreren der vorhergehenden Ansprüche, wobei der Behälterbefestigungsabschnitt (20) und/oder der Griffbefestigungsabschnitt (36) mittels eines Kunststoffschweißverfahrens hergestellt ist.

7. Folienverpackung (10, 100, 110, 150) gemäß einem oder mehreren der vorhergehenden Ansprüche, wobei der Behälterbefestigungsabschnitt (20) und/oder der Griffbefestigungsabschnitt (36) zumindest eine Kunststoffschweißnaht und/oder mehrere Kunststoffschweißpunkte umfasst.

8. Folienverpackung (10, 100, 110, 150) gemäß einem oder mehreren der vorhergehenden Ansprüche, wobei der erste Bogen (14) zumindest eine Faltung (44) umfasst, wobei die Faltung (44) zumindest teilweise im Behälterbefestigungsabschnitt (20) angeordnet ist.

9. Folienverpackung (10, 100, 110, 150) gemäß einem oder mehreren der vorhergehenden Ansprüche, wobei der zweite Bogen (32) zumindest eine erste unbefestigte Kante (50) und eine zweite unbefestigte Kante (52) aufweist, und wobei der Griffbefestigungs-abschnitt (36) zwischen den zwei unbefestigten Kanten (50, 52) ausgebildet ist.

10. Folienverpackung (10, 100, 110, 150) gemäß Anspruch 9, wobei der Griffbefestigungsabschnitt (36) im Wesentlichen mittig zwischen den zwei unbefestigten Kanten (50, 52) des zweiten Bogens angeordnet ist.

11. Folienverpackung (10, 100, 110, 150) gemäß einem oder mehreren der vorhergehenden Ansprüche, wobei der zweite Bogen (32) zumindest eine Faltung oder Biegung (46) umfasst, und wobei der Griffbefestigungsabschnitt (36) von der Faltung oder Biegung (46) beabstandet ausgebildet ist und zumindest eine erste Lage (60) und eine zweite Lage (62) des zweiten Bogens (32) aneinander befestigt.

12. Folienverpackung (10, 100, 110, 150) gemäß einem oder mehreren der vorhergehenden Ansprüche, wobei der Haltegriff (30, 300, 310, 350) zumindest eine zweite Grifföffnung (35) aufweist, wobei die erste Grifföffnung (34) und die zweite Grifföffnung (35) in Bezug auf den Griffbefestigungsabschnitt (36) im Wesentlichen symmetrisch in Bezug zueinander auf dem zweiten Bogen (32) ausgebildet sind.

13. Verwendung einer Folienverpackung (10, 100, 110, 150) gemäß einem oder mehreren der vorhergehenden Ansprüche zur Verpackung von einem einzelnen Hygieneprodukt oder mehreren Hygieneprodukten, wobei die Hygieneprodukte Küchenrollen, Toilettenpapier, Servietten, Taschentücher, Windeln und/oder Binden umfassen.
